# EUROPÄISCHE PATENTSCHRIFT

(11) **EP 0 378 798 B1**
(45) Veröffentlichungstag und Bekanntmachung des Hinweises auf die Patenterteilung: **23.03.1994**
(21) Anmeldenummer: 89122619.3
(22) Anmeldetag: 07.12.1989
(51) Int. Cl.: C12N 9/74, A61K 37/547

(54) **Verfahren zur Herstellung eines hochreinen Thrombinkonzentrates**
Process for preparing a highly purified thrombin concentrate
Procédé de préparation d'un concentré de thrombine très purifié

(30) Priorität: 22.12.1988 DE 3843126
(43) Veröffentlichungstag der Anmeldung: 25.07.1990
(73) Patentinhaber: OCTAPHARMA AG, 8750 Glarus (CH)
(72) Erfinder: Kraus, Michael, Dipl.-Bio., D-6000 Frankfurt am Main 1 (DE); Möller, Wolfgang, Dr. Dipl.-Chem., D-6370 Oberursel (DE)
(74) Vertreter: Meyers, Hans-Wilhelm, Dr.Dipl.-Chem.

(56) Entgegenhaltungen:
- RESEARCH DISCLOSURE, Nr. 269, September 1986, Seiten 564-565, Emsworth, Hampshire, GB; S. BJOERN et al.: "Activation of coagulation factor VII to VIIa"
- J. BIOL. CHEM., Band 246, Nr. 6, März 1971, Seiten 1213-1221, Baltimore, US; A. ENGEL et al.: "Studies of thrombin formation with an insoluble derivative of prothrombin"

## Beschreibung

Gegenstand der Erfindung ist ein einfaches Verfahren zur Isolierung eines Thrombinpräparates hoher spezifischer Aktivität und hoher Ausbeute aus Faktor II enthaltendem Plasma oder Plasmafraktionen.

Die Plasmaprotease Thrombin ist ein multifunktionelles Enzym, das spezifisch mit Proteinen und Zellen reagieren kann. Eine der Hauptfunktionen von Thrombin in Blutplasma ist die Umwandlung von löslichem Fibrinogen in unlösliches Fibrin in dem letzten Schritt in der Blutgerinnungskaskade. Daneben kann Thrombin auch mit den Gerinnungsfaktoren V, VII, IX, XIII und Protein C reagieren und diese aktivieren. Thrombin reagiert ausserdem spezifisch mit Blutplättchen und Endothelzellen und ist in der Lage, Fibroblasten mitogen zu stimulieren. In der Therapie wird Thrombin z.B. zusammen mit Fibrinogen und Faktor XIII in den sogenannten Fibrinklebern zur gezielten Klebung von Verletzungen von Organen, Knochen und Gefässen eingesetzt. Daneben wird Thrombin auch alleine verwendet, z.B. zur Stillung flächenhafter Sickerblutungen oder bei Blutungen in Hohlorganen.

Für alle klinischen Anwendungen, sei es in der Therapie oder der Diagnostik, ist eine hohe Reinheit des Thrombins erforderlich, um unerwünschte Nebeneffekte, z.B. durch andere Proteasen oder Gerinnungsfaktoren, auszuschliessen. In der Literatur sind bisher mehrere Präparationsmethoden für hochgereinigtes Thrombin beschrieben worden. Üblicherweise gehen die beschriebenen Methoden von partiell gereinigtem Thrombin oder von Prothrombinkomplex-Konzentraten aus. Der Hauptanteil der Arbeiten zur Darstellung einer reinen Thrombin-Präparation besteht deshalb in der Herstellung dieser Ausgangsprodukte, während dann die Hochreinigung des Thrombins in ein bis zwei chromatographischen Schritten durchgeführt werden kann. Die spezifische Aktivität des Thrombins im Ausgangsmaterial für diese chromatographische Hochreinigung war bisher von der Reinheit des Faktors II vor der Aktivierung zu Thrombin abhängig und lag zwischen 20-250 NIH-E/mg Protein [Zur Übersicht: Machovich R. (Ed.), Boca Raton: Thrombin Vol. 1, S.1-160 (1984)].

Nach dem Stand der Technik war es bis heute nicht möglich, Thrombin mit einem einfachen einstufigen Verfahren mit hoher Ausbeute aus Plasma zu isolieren. Das Standardverfahren zur Herstellung eines Thrombin-Konzentrates besteht zur Zeit aus der Adsorption der PPSB-Faktoren II, VII, IX und X aus plasma mit einem DEAE-Träger, der Elution von Faktor II und der anschliessenden Aktivierung des Faktor II zu Thrombin mit anschliessender Weiteraufreinigung. Die Ausbeuten für Faktor II bei dem ersten Schritt dieses Verfahrens liegen bei 40-60% der Ausgangsaktivität im Plasma. Nach der Aktivierung des Faktors II und der weiteren Aufreinigung des Thrombins liegen die Ausbeuten, abhängig von den verwendeten Verfahren, bei 20-40 NIH-E Thrombin pro Einheit Faktor II im Ausgangsplasma und damit bei rund 20000 bis 40000 NIH-E Thrombin pro Liter Plasma.

Die bisher bekannten Methoden der Isolierung von Thrombin aus Plasma konnten auf eine vorherige Präparation des Faktor II nicht verzichten, da eine Aktivierung des Faktor II im Plasma zu einer Gerinnung des Fibrinogens zu Fibrin führt und damit gleichzeitig aus Plasma Serum gebildet wird. Abgesehen von Verlusten von Thrombin durch Adsorption an das Fibrin wäre die Weiteraufarbeitung des Serums zu anderen Gerinnungsfaktoren oder den anderen therapeutisch nutzbaren Plasmaproteinen erschwert bzw. unmöglich. Im Sinne einer optimalen Ausnutzung des wertvollen Rohstoffs Humanplasma ist es aber gerade geboten, nur solche Präparationsmethoden anzuwenden, die die Reinigungsverfahren für andere Plasmaproteine möglichst wenig beeinflussen.

Der vorliegenden Erfindung lag danach die Aufgabe zugrunde, ein einfaches Verfahren zur Isolierung von Thrombin aus Plasma mit hoher spezifischer Aktivität und hoher Ausbeute zu finden.
Diese Aufgabe wird erfindungsgemäss dadurch gelöst, dass man den Faktor II aus Plasma oder Plasmafraktionen an einen festen Träger adsorbiert, den Faktor II anschliessend auf der Matrix zu Thrombin aktiviert und spezifisch eluiert.

Überraschenderweise wurde gefunden, dass man durch die Aktivierung des auf der Matrix adsorbierten Faktor II zu Thrombin diesen mit einer höheren Ausbeute und mit einer wesentlich höheren Reinheit gewinnen kann, als wenn man den Faktor II erst eluiert, dann zu Thrombin aktiviert und anschliessend weiter aufreinigt.
Die durch dieses Verfahren erreichbaren Ausbeuten liegen bei 70-90 NIH-E Thrombin pro eingesetzter Einheit Faktor II und damit bei 70000 bis 90000 NIH-E Thrombin pro Liter Ausgangsplasma bei einer spezifischen Aktivität von 800-1400 NIH-E pro mg Protein.

Das erfindungsgemässe Verfahren zur Isolierung von Thrombin aus Plasma lässt sich hervorragend in die bisherigen Fraktionierschemata einpassen, da diese meist einen Schritt zur Adsorption der PPSB-Faktoren aus Plasma enthalten. Der Unterschied zur Isolierung der PPSB-Faktoren besteht hier in den unterschiedlichen Elutionsbedingungen für das Adsorbents.

Ein erfindungsgemässes Präparat kann auf folgendem Wege hergestellt werden:
Eine Faktor II-haltige Lösung, vorzugsweise Humanplasma oder eine Plasmafraktion, wird an eine Matrix, vorzugsweise einen Anionenaustauscher oder einen Affinitätsträger, adsorbiert. Die Adsorption erfolgt unter an sich bekannten und dem Stand der Technik entsprechenden Bedingungen, z.B. niedriger Ionenstärke von 10 mS bis 150 mS und einem pH von 6,0 bis 8,5 bei der Verwendung von Fraktogel DEAE-TSK (Merck, Darmstadt).
Nicht gebundene Proteine werden mit einem Citrat-Puffer und dann das Citrat, welches die Aktivierung stören würde, mit einer niedermolekularen Kochsalzlösung von der Matrix gewaschen. Zur Elution wird ein Puffer auf die Matrix aufgebracht, der einen Aktivator für Faktor II, z.B. Ca²⁺-Ionen, Ca²⁺-Ionen und Thromboplastin oder Faktor Xa, enthält. Vorzugsweise wird der Faktor II nur mit Ca²⁺-Ionen zu Thrombin aktiviert, indem man die Matrix für 10 Minuten bis 5 Stunden mit einer Ca²⁺-Ionenlösung mit einer Konzentration von vorzugsweise 20 bis 30 mM Ca eluiert. Eine besonders hohe Anreicherung des Thrombins kann durch das Inkubieren der Säule mit einer Ca²⁺-Ionenlösung für 10 Minuten bis 5 Stunden und anschliessendem Eluieren erreicht werden.

Die Fraktionen mit einer spezifischen Aktivität von mehr als 400 NIH-E pro mg Protein werden gepoolt. Die Thrombinaktivität wird dabei über die Umwandlung von Fibrinogen zu Fibrin (R.L. Lundblad, Biochemistry 10 (13), 2501-2506, 1971) und durch die Umsetzung eines chromogenen Substrates (Chromozym TH, Boehringer) bestimmt. Der Proteingehalt der Fraktionen wird nach der Methode von Bradford (M.M.Bradford, Anal. Biochem. 72, 248-254, 1976) gemessen. Die Thrombinfraktion wird durch an sich bekannte Massnahmen, wie z.B. Ultrafiltration, auf einen Thrombingehalt von mehr als 100 NIH-E pro ml konzentriert und sterilfiltriert. Die spezifische Aktivität liegt üblicherweise bei rund 800 bis 1400 NIH-E pro mg Protein.
Wird eine noch höhere Reinheit gewünscht, kann durch eine direkte Adsorption der von der Matrix eluierten Thrombinlösung an einen Kationenaustauscher, wie z.B. SP-Trisacryl (IBF), Fraktogel TSK-Sulfat (Merck) oder SP-Sephadex C50 (Pharmacia) die spezifische Aktivität des Thrombins leicht auf über 2000 NIH-E pro mg Protein gesteigert werden.

Vor oder nach der Isolierung des Thrombins aus Plasma oder Plasmafraktionen kann eine Sterilisationsmassnahme zur Inaktivierung humanpathogener Viren, wie Hepatitis Non A NonB oder HIV, z.B. durch Behandlung mit β-Propiolacton/UV-Strahlung, durch Behandlung mit Tri-n-butylphosphat/Detergens oder durch Erhitzen durchgeführt werden.

Die folgenden Beispiele erläutern das erfindungsgemässe Verfahren weiter.

### Beispiel 1

Eine 50 ml Säule mit Fraktogel TSK-DEAE wurde mit 10 mM Citrat, pH 7,0, equilibriert. 1000 ml Citrat-Plasma wurden mit einer Flussrate von 750 ml/h auf die Säule aufgetragen. Anschliessend wurde das Gel mit 100 ml 10 mM Citrat und mit 100 ml 75 mM NaCl gespült. Das Thrombin wurde mit 25 mM CaCl₂ bei einer Flussrate von 100 ml/h für 4 h eluiert. Die spezifische Aktivität des Thrombin-Eluates lag bei 1000 NIH-Einheiten pro mg Protein. Aus 1000 ml Plasma, das 1040 Einheiten Faktor II enthielt, konnten 90500 NIH-E Thrombin gewonnen werden. Dies entspricht einer Ausbeute von 87 NIH-E Thrombin pro Einheit Faktor II im Ausgangsplasma.

### Beispiel 2

Jeweils 1000 ml Citrat-Plasma wurden analog zum Beispiel 1 über verschiedene Träger chromatographiert.
Die Ergebnisse sind in der Tabelle wiedergegeben:

**Tabelle**

| Träger | spez. Akt. (NIH-E-Thrombin/mg Protein) | Gesamtausbeute Thrombin (NIH-E) |
|---|---|---|
| DEAE-Sephadex A50 | 246 | 85300 |
| QA-Trisacryl LS | 863 | 63000 |
| DEAE-Trisacryl LS | 950 | 91000 |
| Fraktogel TSK-Amino | 1400 | 86900 |

### Beispiel 3

1000 ml Kryo-poor Plasma wurden analog zu Beispiel 1 über Fraktogel TSK-DEAE chromatographiert.

Die spezifische Aktivität des Thrombin-Eluates lag bei 950 NIH-Einheiten pro mg Protein bei einer Gesamtausbeute von 72600 NIH-Einheiten Thrombin.

### Beispiel 4

100 ml Thrombin-Eluat aus Beispiel 1 wurden sofort auf eine 15 ml Säule mit dem Kationenaustauscher Fraktogel TSK-Sulfat aufgetragen, die vorher mit 50 mM NaCl equilibriert wurde.
Das Gel wurde mit 50 ml 50 mM NaCl und mit 100 ml 75 mM Phosphat-Puffer, pH 6,5, gewaschen. Das Thrombin wurde dann mit 250 mM Phosphat-Puffer, pH 6,5, eluiert.
Die Ausbeute bei diesem Versuch betrug 86%, bezogen auf das Ausgangseluat, und 78000 NIH-E Thrombin, bezogen auf einen Liter Ausgangsplasma. Die spezifische Aktivität des Thrombins konnte auf 2400 NIH-E/mg Protein gesteigert werden.

## Patentansprüche

1. Verfahren zur Isolierung von Thrombin aus Faktor II-haltigen Lösungen, dadurch gekennzeichnet, dass der Faktor II an einen festen Träger mit geringerer Affinität für Thrombin als für Faktor II adsorbiert, der Faktor II auf der Matrix zu Thrombin aktiviert und dieses anschliessend eluiert wird.

2. Verfahren nach Anspruch 1, dadurch gekennzeichnet, dass als fester Träger ein Anionenaustauscher verwendet wird.

3. Verfahren nach Anspruch 1, dadurch gekennzeichnet, dass als fester Träger ein Affinitätsträger für Faktor II verwendet wird.

4. Verfahren nach Anspruch l oder 2, dadurch gekennzeichnet, dass man als festen Träger DEAE-Gruppen oder QAE-Gruppen tragende Polymere vom Typ eines Copolymerisates von Glycidylmethacrylat, Pentaerythrol-Dimethacrylat und Polyvinylalkohol oder vom Typ eines Copolymerisates von N-Acryloyl-2-amino-2-hydroxymethyl-1,3-propandiol oder Polymere aus Kohlehydraten verwendet.

5. Verfahren nach Anspruch 1, dadurch gekennzeichnet, dass man als festen Träger eine 2-Hydroxy-Aminopropyl-Gruppen tragende Matrix verwendet.

6. Verfahren nach Anspruch 5, dadurch gekennzeichnet, dass man als 2-Hydroxy-Aminopropyl-Gruppen tragende Matrix Polymere vom Typ eines Copolymerisates von Glycidylmethacrylat, Pentaerythrol-Dimethacrylat und Polyvinylalkohol verwendet.

7. Verfahren nach Anspruch 1 bis 6, dadurch gekennzeichnet, dass man den Faktor II auf der Matrix mit Calcium-Ionen aktiviert.

8. Verfahren nach Anspruch 7, dadurch gekennzeichnet, dass man den Faktor II auf der Matrix mit Calcium-Ionen in einer Konzentration von 20 bis 30 mM aktiviert.

9. Verfahren nach Anspruch 1 bis 8, dadurch gekennzeichnet, dass die Faktor II-haltige Lösung Plasma, eine Plasmafraktion oder Kulturmedium von Faktor II synthetisierenden Zellen ist.

10. Verfahren nach Anspruch 1 bis 9, dadurch gekennzeichnet, dass man die Faktor II-haltige Lösung Vor der Adsorption an den Träger oder die Thrombinlösung nach der Elution einem virusinaktivierenden Schritt unterzieht.

## Claims

1. A process for isolating thrombin from solutions containing factor II, characterized in that the factor II adsorbs to a solid support having lower affinity for thrombin than for factor II, the factor II on the matrix is activated to give thrombin which subsequently is eluted.

2. The process according to claim 1, characterized in that an anion exchanger is used as the solid support.

3. The process according to claim 1, characterized in that a factor II affinity support is used as the solid support.

4. The process according to claim 1 or 2, characterized in that polymers bearing DEAE groups or QAE groups and being of a type of a copolymerizate of glycidyl methacrylate, pentaerythritol dimethacrylate and polyvinyl alcohol or of a type of a copolymerizate of N-acryloyl-2-amino-2-hydroxymethyl-1,3-propanediol, or polymers of carbohydrates are used as the solid support.

5. The process according to claim 1, characterized in that a matrix bearing 2-hydroxyaminopropyl groups is used as the solid support.

6. The process according to claim 5, characterized in that polymers of a type of a copolymerizate of glycidyl methacrylate, pentaerythritol dimethacrylate and polyvinyl alcohol are used as the matrix bearing 2-hydroxyaminopropyl groups.

7. The process according to claims 1 to 6, characterized in that said factor II is activated on the matrix using calcium ions.

8. The process according to claim 7, characterized in that said factor II is activated on the matrix using calcium ions at a concentration of from 20 to 30 mM.

9. The process according to claims 1 to 8, characterized in that the solution containing factor II is plasma, a plasma fraction or a culture medium of cells synthesizing factor II.

10. The process according to claims 1 to 9, characterized in that the solution containing factor II prior to adsorption on the support, or the thrombin solution subsequent to elution is subjeceted to a step of virus inactivation.

## Revendications

1. Procédé d'isolement de la thrombine à partir de solutions contenant du facteur II, caractérisé en ce que le facteur II est adsorbé sur un porteur solide ayant une affinité plus faible pour la thrombine que pour le facteur II, en ce que le facteur II sur la matrice est activé en thrombine et en ce que celle-ci est enfin éluée.

2. Procédé selon la revendication 1, caractérisé en ce qu'un échangeur d'anions est utilisé comme porteur solide.

3. Procédé selon la revendication 1, caractérisé en ce qu'un porteur d'affinité pour le facteur II est utilisé comme porteur solide.

4. Procédé selon la revendication 1 ou 2, caractérisé en ce qu'on utilise comme porteur solide des polymères portant des groupements DEAE ou des groupements QAE du type d'un copolymère de méthacrylate de glycidyle, de diméthacrylate de pentaérythritol et d'alcool polyvinylique, ou du type d'un copolymère N-acryloyl-2-amino-2-hydroxyméthyl-1,3-propanediol ou des polymères de glucides.

5. Procédé selon la revendication 1, caractérisé en ce qu'on utilise comme porteur solide une matrice portant des groupements 2-hydroxy-aminopropyle.

6. Procédé selon la revendication 5, caractérisé en ce qu'on utilise comme matrice portant des groupements 2-hydroxy-aminopropyle des polymères du type d'un copolymère de méthacrylate de glycidyle, de diméthacrylate de pentaérythritol et d'alcool polyvinylique.

7. Procédé selon les revendications 1 à 6, caractérisé en ce qu'on active le facteur II sur la matrice avec des ions calcium.

8. Procédé selon la revendication 7, caractérisé en ce qu'on active le facteur II sur la matrice avec des ions calcium à une concentration de 20 à 30 mM.

9. Procédé selon les revendications 1 à 8 caractérisé en ce que la solution plasmatique contenant le facteur II est une fraction de plasma ou un milieu de culture de cellules synthétisant le facteur II.

10. Procédé selon les revendications 1 à 9, caractérisé en ce que la solution contenant le facteur II ou la solution de thrombine subissent - avant l'adsorption sur le porteur ou après l'élution respectivement - un traitement d'inactivation virale.
